Europäisches Patentamt

European Patent Office

Office européen des brevets

(11) Publication number: **0 433 242 A1**

## EUROPEAN PATENT APPLICATION

(21) Application number: 90830455.3

(22) Date of filing: **15.10.90**

(51) Int. Cl.5: **A61K 9/127, A61K 39/00**

The title of the invention has been amended (Guidelines for Examination in the EPO, A-III, 7.3).

(30) Priority: **16.10.89 IT 4845889**

(43) Date of publication of application:
**19.06.91 Bulletin 91/25**

(84) Designated Contracting States:
**AT BE CH DE DK ES FR GB GR LI LU NL SE**

(71) Applicant: **FONDAZIONE ANDREA CESALPINO**
**169 Via Sicilia**
**I-00187 Roma RM(IT)**

(72) Inventor: **Balsano, Francesco**
**44 Via Clitunno**
**I-00198 Roma RM(IT)**
Inventor: **Barnaba, Vincenzo**
**86 Via Mascagni**
**I-00199 Roma RM(IT)**

(74) Representative: **Di Cerbo, Mario et al**
**Società Italiana Brevetti S.p.A., Piazza di Pietra 39**
**I-00186 Roma(IT)**

(54) Vaccines and process for their preparation.

(57) The process for the preparation of vaccines according to the invention is essentially characterized by the fact of introducing, into particles of lipoprotein membranes, antigens inducing a protective immune response. The lipoprotein membranes can be natural, as for example the liposomes, and/or synthetic. Antigens inducing a protective immune response can be, for example, proteins of hepatitis B virus (HBV), AIDS virus (HIV), malaria plasmodium or toxoplasma. The invention extends also to the vaccines characterized by the fact of consisting in, or including, particles constituted by lipoprotein membranes containing antigens inducing a protective immune response.

EP 0 433 242 A1

# PROCESS FOR PREPARING VACCINES CAPABLE OF GENERATING NOT ONLY THE IMMUNE RESPONSE OF T HELPER LYMPHOCYTES, BUT ALSO AN EFFECTIVE RESPONSE OF CYTOTOXIC T LYMPHOCYTES; AND VACCINES WITH THE ABOVE CHARACTERISTICS

### DESCRIPTION

The present invention refers to a process for the preparation of vaccines consisting in, or including, particles constituted by lipoprotein membranes containing antigens that induce a protective immune response. These particles, eventually together with other ordinary substances for vaccine preparation, are capable of generating not only an immune response of T helper lymphocytes (class II-restricted) but also an effective response of cytotoxic T lymphocytes (class I-restricted). It must be emphasized that these last cells are fundamental for the clearance of cells infected by pathogenic agents.

As it is known, the recognition of antigen by T lymphocytes can be differentiated from the recognition by B lymphocytes for two main reasons: a) T cells do not recognize, as B lymphocytes, the native antigen in its conformation, but only peptidic fragments derived from its processing by antigen presenting cells (APC); b) such peptides are recognized only if associated with other membrane molecules of APC, constituted by the products of major histocompatibility complex (MHC) genes.

T lymphocytes with CD4+ phenotype recognize the antigen in association with class II MHC molecules. On the contrary, T lymphocytes with CD8+ phenotype recognize the antigen in association with class I NHC molecules.

The experimental evidence suggests thee existence of two separate pathways of processing and presentation of antigen, that lead to the selective association of cellular endogenous antigens to class I molecules, and of exogenous antigens to class II molecules. Consequently, CD4+ class II-restricted T cells are usually induced by exogenous soluble antigens, while CD8+ class I-restricted T cells are generally induced by endogenously synthesized antigens, such as those expressed by virus infected cells, or by cellular self antigens.

Summarizing, exogenous antigens, which enter into the APC through a fluid phase mechanism or by receptor-mediated endocytosis, are processed by acid proteases at the endosomal level and seem to have a selective access to class II molecules, as a result of a vescicular fusion at the level of trans- or post-Golgi region. On the contrary, peptides derived from proteins synthesized by the cells themselves, would be processed in the cytosol (probably from enzymes of cis-medial-Golgi but not of trans-Golgi) and could preferentially interact with class I molecules at the level of endoplasmic reticulum or of the cis-Golgi region.

All that has a precise biological sense: for example, during a viral infection, cytotoxic class I-restricted T cells will destroy the virus infected cells, while helper class II-restricted T cells will induce an antibody response to neutralize exogenous viral particles or toxins.

It has now been unexpectedly found that utilizing the process of the present invention it is possible to prepare vaccines capable not only of an effective class II-restricted helper T lymphocytes response, but also a response of class I-restricted cytotoxic T lymphocytes. The present invention therefore refers to a process for the preparation of vaccines, essentially characterized by the fact of introducing, into lipoprotein membrane particles, antigens inducing a protective immune response.

Lipoprotein membranes can be natural membranes, as for example the liposomes, and/or synthetic membranes.

The antigens that determine the protective immune response can be, for example, proteins of hepatitis B virus (HBV) or AIDS virus (HIV).

In these cases, the above-mentioned antigens can be recombinant proteins or synthetic peptides of the virus under consideration.

The antigens that determine the protective immune response can also be, for example, proteins of the malaria plasmodium or of toxoplasma.

The present invention is not limited to the process for vaccine preparation, but extends to the vaccines per se.

Subject matter of the present invention are therefore also vaccines characterized by the fact of consisting in, or including, particles consituted by lipoprotein membranes containing antigens capable of inducing a protective immune response. These vaccines are capable to generate not only the immune response of T helper (class II-restricted) lymphocytes, but also an effective response of cytotoxic (class I-restricted) T lymphocytes.

The lipoprotein membranes can be natural, as for example the liposomes, and/or synthetic.

As previously seen, the antigens that determine the protective immune response can be, for example, proteins of hepatitis B virus (HBV) or of AIDS virus (HIV). In these cases, the above-mentioned antigens can be recombinant proteins or synthetic peptides of the virus under consideration.

The antigens that determine the protective immune response can also be, for example, proteins of the malaria plasmodium or of toxoplasma.

The vaccines according to the present invention can obviously contain also substances usual in the preparation of vaccines.

So far, a general description of the invention forming the subject matter of the present patent has been given. To better understand its purposes, characteristics and advantages, a more detailed description will now be given with the help of the following example.

The example has only an explanatory function. Indeed the scope of the invention is substantially defined only by the following claims.

Example

A few very rare antigens, as for example the hepatits B virus envelope (subsequently indicated as hepatitis B virus or HBV) can be presented to class I-restricted T cells. In particular, the processing pathway of this exogenous antigen necessary for tlhe binding to class I molecules would not be the endosomial one, but another processing pathway not yet well elucidated.

It has therefore been investigated on the possibility that exogenous antigens could be processed and presented to class II-restricted T lymphocytes, when directly introduced into the cytosol, following a non-endosomal processing pathway that lead the peptidic fragments to bind class I molecules. For this purpose, there have been prepared particles consituted by lipoprotein membranes containing the HBV antigenic envelope proteins (pre-S1, pre-S2, S) to verify if such particles, by virtue of the fact that they contain lipoprotein membranes, can enter into the APC through a fusion mechanism with the cellular membrane, introducing the HBV antigens into the cytosol.

Therefore, there have been constructed two types of particles: a) particles constituted by liposomes, with the HBV envelope proteins assembled both inside and outside of the liposomes; b) particles constituted by liposomes containing the viral proteins only inside.

At this point, there have been generated T clones, specific for B virus envelope protein, by the limiting dilution technique, both from liver infiltrating lymphocytes of B virus chronic hepatitis patients, and by the peripheral lymphocytes obtained from HBV vaccine recipients.

In particular, peripheral mononuclear cells from vaccine recipients have been cultured at a concentration of 4x10$^5$ cells in a total volume of 200 $\mu$l in 96 well flat-bottomed microplates. Furthermore, lymphocytes isolated from liver biopsies of HBV chronic hepatitis patients were cultured with the antigen, together with irradiated peripheral autologous mononuclear cells, used as APC. After 7 days, interleukin 2 (IL2) has been added, and, after further 15 days, T blasts cloned at 1-0.3 cells/well with antigen, IL2 and irradiated autologous APC. After 10-15 days, cultures containing proliferating blasts have been further expanded with IL2, and stimulation with antigen was periodically performed, to obtain an adequate number of T lymphocytes derived from a single precursor, to perform specificity and HLA-restriction studies.

In this way, there have been obtained both class II-restricted T and class I-restricted T clones. These antigen-specific T clones have been so cultured with the two types of particles, artificially prepared as described above. In particular, both class II- and class I-restricted T clones were capable to recognize the liposomes containing the viral proteins both inside and outside. On the contrary, only class I-restricted clones recognized liposomes containing the viral proteins only inside.

Class II-restricted T lymphocytes could not recognize this second type of particle because the latter, not containing viral antigen outside, cannot enter into the APC through the classic mechanism of endocytosis, that, as already said, lead to a selective association with class II molecules.

Studies with APC fixation have shown that the two forms of particles need to be catched and processed before being presented to T lymphocytes. These results demonstrate that the ability of an exogenous antigen to be recognized by class I-restricted T cells depends on the fact that it, as being contained in liposomes, can in turn enter into APC cellular membrane and be introduced into the cytosol, where it will interact with class I molecules.

The vaccine against HBV hepatitis, prepared with the criteria described above, presents, with respect to traditional vaccines against HBV hepatitis, the following advantages:
- it can avoid the use of potentially infecting plasma or plasma- and hemo-derivatives;
- it surely evokes a cytotoxic T lymphocyte response (traditional vaccines activate only T helper cells);
- it consequently avoids the vaccine non-responder state; and
- it vehiculates the immunodominant epitopes (in this way, the synthetic peptides recognized by helper and/or cytotoxic T cells are introduced into liposomes).

**Claims**

1. A process for the preparation of vaccines, characterized essentially by the fact of introducing, into lipoprotein membrane particles, antigens that determine the protective immune response, the particles so obtained being capable of generating not only the immune response of T helper (class II-restricted) lymphocytes, but also an effective response of cytotoxic (class I-restricted) T lymphocytes.

2. The process for the preparation of vaccines as per claim 1, in which the lipoprotein membranes are totally or partically natural.

3. The process for the preparation of vaccines as per claim 2, in which the natural lipoprotein membranes are the liposomes.

4. The process for the preparation of vaccines as per claim 1, in which the lipoprotein membranes are totally or partially synthetic.

5. The process for the preparation of vaccines as per any of the preceding claims, in which the antigens that determine the protective immune response are HBV proteins.

6. The process for the preparation of vaccines as per claims 1 to 4, in which the antigens that determine the protective immune response are proteins of AIDS virus (HIV).

7. The process for the preparation of vaccines as per claim 5 or 6, in which the antigens that determine the protective immune response are recombinant proteins of the virus.

8. The process for the preparation of vaccines as per claim 5 or 6, in which the antigens that determine the protective immune response are synthetic peptides of the virus.

9. The process for the preparation of vaccines as per claims 1 to 4, in which the antigens that determine the protective immune response are proteins of malaria plasmodium.

10. The process for the preparation of vaccines as per claims 1 to 4, in which the antigens that determine the protective immune response are proteins of toxoplasma.

11. Vaccines, characterized by the fact of consisting in, or including, particles constituted by lipoprotein membranes containing antigens capable of inducing a protective immune response, these vaccines being capable to generate not only the immune response of T helper (class II-restricted) lymphocytes, but also an effective response of cytotoxic (class I-restricted) T lymphocytes.

12. Vaccines as per claim 11, in which the lipoprotein membranes are totally or partially natural.

13. Vaccines as per claim 12, in which the natural lipoprotein membranes are the liposomes.

14. Vaccines as per claim 11, in which the natural lipoprotein membranes are totally or partially synthetic membranes.

15. Vaccines as per any claim 11 to 14, in which the antigens that determine the protective immune response are proteins of hepatitis B virus (HBV).

16. Vaccines as per any claim 11 to 14, in which the antigens that determine the protective immune response are proteins of AIDS virus (HIV).

17. Vaccines as per claim 15 or 16, in which the antigens that determine the protective immune response are recombinant proteins of the virus.

18. Vaccines as per claim 15 or 16, in which the antigens that determine the protective immune response are synthetic peptides of the virus.

19. Vaccines as per claims 11 to 14, in which the antigens that determine the protective immune response are protein of malaria plasmodium.

20. Vaccines as per claims 11 to 14, in which the antigens that determine the protective immune response are proteins of toxoplasma.

21. Vaccines as per any claim 11 to 20, in which substances usual in the preparation of vaccines are present.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 277 930 (UNIVERSITE LIBRE DE BRUXELLES) * Page 2, lines 30-50; page 7, example 7; claims * | 1-4,6, 11-14,16, 21 | A 61 K 9/127 A 61 K 39/00 |
| A | US-A-4 235 877 (FULLERTON) * Columns 3,4; examples 3,5 * | 5,15 | |
| A | EP-A-0 306 912 (ALBANY MEDICAL COLLEGE) * Page 2, line 1 - page 3, line 43; page 6, line 10 - page 10, line 57; page 20, examples 6,7 * | 7-9,17-20 | |
| A | EP-A-0 276 783 (MAX-PLANCK-GESELLSCHAFT ZUR FÖRDERUNG DER WISSENSCHAFTEN e.V.) * Page 3, lines 36-44 * | 2-4,12-14 | |
| A | FR-A-2 325 387 (THE WELLCOME FOUNDATION LTD) * The whole document * | 1,11 | |
| A | EP-A-0 047 480 (INSTITUT ARMAND FRAPPIER) * Page 1, line 1 - page 4, line 7 * | 1,11 | |

TECHNICAL FIELDS SEARCHED (Int. Cl.5)

A 61 K

The present search report has been drawn up for all claims

| Place of search | Date of completion of search | Examiner |
|---|---|---|
| The Hague | 01 February 91 | BENZ K.F. |